# EUROPÄISCHE PATENTSCHRIFT

(11) **EP 0 958 780 B1**
(45) Veröffentlichungstag und Bekanntmachung des Hinweises auf die Patenterteilung: **07.10.2009**
(21) Anmeldenummer: 99810408.7
(22) Anmeldetag: 07.05.1999
(51) Int. Cl.: A61B 5/00

(54) **Sensorsystem mit permanent implantiertem Zugang**
Sensor system with permanently implanted access
Système à capteur avec accès permanemment implanté

(30) Priorität: 20.05.1998 DE 19822711
(43) Veröffentlichungstag der Anmeldung: 24.11.1999
(73) Patentinhaber: F. Hoffmann-La Roche AG, 4070 Basel (CH); Roche Diagnostics GmbH, 68305 Mannheim (DE)
(72) Erfinder: Michel, Willi, 3400 Burgdorf (CH); Haueter, Ulrich, 3506 Grosshöchstetten (CH); Frei, Thomas, 3452 Lützelflüh (CH)
(74) Vertreter: Gassenhuber, Andreas

(56) Entgegenhaltungen:
- EP-A- 0 534 074
- WO-A-95/28878
- US-A- 4 805 624
- US-A- 5 441 481
- US-A- 5 474 552

## Beschreibung

Die Erfindung betrifft eine Vorrichtung zur Erfassung der Konzentration bzw. des Vorhandenseins von Stoffen in Körperflüssigkeiten.

Es gibt im medizinischen Bereich bisher mehrere Verfahren, um die Konzentration bzw. das Vorhandensein von Stoffen in Körperflüssigkeiten zu erfassen. Bei der Feststellung der Blutzuckerkonzentration besteht zum einen die Möglichkeit, die Haut eines Patienten, beispielsweise am Finger, etwas aufzuritzen und mit Hilfe von Teststreifen den Blutzuckergehalt aus dem so gewonnenen Blut und/oder der so gewonnen Zwischenzellflüssigkeit zu ermitteln. Ferner wird von der Möglichkeit Gebrauch gemacht, dünne Nadeln zur Entnahme einzustechen oder einen Sensor an eine Meßstelle innerhalb des Körpers zu führen.

Solche invasiven Techniken haben immer den Nachteil, daß sie für den Patienten unangenehm sind. Nadelsensoren versagen außerdem, da sie schon nach kürzester Zeit (etwa nach einem halben Tag) vom Körper angegriffen bzw. abgestoßen werden.

Nichtinvasive Techniken, wie beispielsweise die Konzentrationsfeststellung über Durchleuchtungstechniken, sind zur Zeit technisch noch nicht ausreichend ausgereift, um genügend exakte Werte zu erhalten. Portkörper, die subkutan oder perkutan implantiert werden und der Verabreichung von Arzneimitteln über Schlauchkanäle dienen, sind beispielsweise aus der US-A-5,306,255 und der EP-B-0 302 076 bekannt.

Aus der EP 0 534 074 A1 ist eine Anordnung zur kontinuierlichen Bestimmung der Konzentration von Inhaltsstoffen in Körperflüssigkeiten bekannt, wobei eine im Körpergewebe implantierbare Dialysesonde vorgesehen ist. Diese Dialysesonde besteht im Wesentlichen aus einer an ihrem proximalen und distalen Ende mittels eines Stopfens verschlossenen Zylinderhülse, die im Bereich ihres distalen Endes eine Dialysemembran aufweist. Dabei ist die Dialysemembran so ausgebildet, dass das durch den Zuflusskanal der Dialysesonde einströmende Perfusat durch die Membran hindurch mit Inhaltsstoffen aus dem interzellularen Gewebe befrachtet wird, wobei die Porosität der Dialysemembran so gewählt wird, dass größere Inhaltsstoffe zurückgehalten werden und wobei die Porenweite im Bereich von 0,01 bis 0,03 Mikrometer gewählt wird.

Die WO 95/ 28878 offenbart eine Subkutan im Körper eines Patienten angebrachte Sensoreinheit ohne physische Verbindung zu einem Bereich außerhalb des Körpers, welche lediglich Daten mit anderen Einheiten austauschen kann.

Es ist die Aufgabe der vorliegenden Erfindung, eine Vorrichtung zur Erfassung der Konzentration bzw. des Vorhandenseins von Stoffen in Körperflüssigkeiten bereitzustellen, mit denen die oben angeführten Nachteile des Standes der Technik soweit als möglich überwunden werden. Insbesondere sollen Unannehmlichkeiten für Patienten vermieden werden.

Bei einem Verfahren zur Erfassung der Konzentration bzw. des Vorhandenseins von Stoffen in Körperflüssigkeiten wird zur Analyse der Flüssigkeiten über einen permanent im Körper implantierten Zugang zum Körperinneren auf die vorhandene Flüssigkeit zugegriffen .

Viele Patienten, die aufgrund einer zu niedrigen oder erhöhten Konzentration bestimmter Substanzen in ihren Körperflüssigkeiten auf eine regelmäßige Arzneimittelzufuhr angewiesen sind, bekommen solche Arzneimittel über künstliche Zugänge zum Körperinneren verabreicht. Das Verfahren zielt nunmehr darauf ab, permanent im Körper implantierte Zugänge für den Zugriff auf Körperflüssigkeiten zu verwenden. Solche permanent im Körper implantierte Zugänge, die Schnellwechseleinheiten umfassen können, beispielsweise eine eingefaßte Verschlußmembran, münden meist an solchen Körperstellen, wo eine Verstopfung der Mündungen über längere Zeit nicht auftritt oder sie sind so konstruiert, daß Angriffe oder Abstoßungsreaktionen durch die Körperzelle ihre Funktion zumindest im vorbestimmten Nutzungszeitraum nicht beeinträchtigen können. Dieser Vorteil kann nun erfindungsgemäß auch für den Zugriff auf die Körperflüssigkeiten mittels solcher Zugänge genutzt werden. Dies bedeutet, daß solche permanenten Zugänge es gestatten, zu Analysezwecken auch über längere Zeit auf Flüssigkeiten zugreifen zu können.

Der Zugriff erfolgt deshalb vorteilhafterweise über eine permanent implantierte Vorrichtung zur Verabreichung von Arzneimitteln und bei einer besonders bevorzugten Ausführungsform des Verfahrens über einen in der Haut implantierten Portkörper mit einer sich ins Körperinnere erstreckenden Schlauchanordnung.

Insbesondere eignen sich Portkörper, die zur Insulinverabreichung als permanenter Zugang, bevorzugt zur Umbilicalvene in der Bauchhöhle im Körper implantiert sind.

Der Bauchraum, das Peritoneum und auch die Umbilicalvene haben sich als vorteilhafte Orte zur Verabreichung von Insulin erwiesen, da das Insulin dort schneller resorbiert wird als beispielsweise bei einer subkutanen Zuführung. Da der Insulinverbrauch in den Körperzellen selbst stattfindet, erscheint die Zellflüssigkeit aber auch als sehr geeigneter Ort zur Messung des Blutzuckergehalts, da hier praktisch direkt am Verbrauchsort ein zentraler Durchschnittswert gemessen wird. Demnach eignen sich Portkörper zur Insulinverabreichung in den Bauchraum besonders gut auch als erfindungsgemäße Zugriffsstellen auf die zu analysierende Körperflüssigkeit.

Ferner sind solche Portkörper zwar invasive Vorrichtungen, jedoch genügt hier eine einmalige Implantation, so daß der Patient bei nachfolgenden Zugriffen auf seine Körperflüssigkeit über solche Portkörper keine Unannehmlichkeiten mehr verspürt.

Eine Ausführungsform eines Verfahrens zeichnet sich dadurch aus, daß ein Prüfsensor über den Zugang zur Erfassung der Konzentration bzw. des Vorhandenseins der Stoffe in das Körperinnere eingeführt und die Erfassung dort vorgenommen wird. Hier eröffnet sich die Möglichkeit einer kontinuierlichen oder intermittierenden Messung, bei der der Prüfsensor am Meßort im Körperinneren verbleibt, wobei er bei der Verwendung eines Portkörpers mit Schlauchanordnung insbesondere über einen separaten Schlauch eingebracht wird.

Eine weitere Möglichkeit, ein Erfassungsverfahren mit einem im Körperinneren verbleibenden Prüfsensor durchzuführen, besteht darin, daß über den Zugang Körperflüssigkeit im Körperinneren entnommen und an einer Stelle analysiert wird, die vom Entnahmepunkt entfernt liegt. Beispielsweise wird die Körperflüssigkeit noch im Körper, bevorzugt an einer Zwischenstelle der Schlauchanordnung mittels eines Sensors analysiert und zu diesem Zweck zu dieser Stelle hin angesaugt. Der Vorteil der Verwendung einer solchen Zwischenstelle als Meßort liegt insbesondere darin, daß mögliche Ablagerungen, die an der Mündung des Schlauches, über den der Zugriff erfolgt, entstehen können, dann keinen negativen Einfluß auf die Meßgenauigkeit haben, wenn der Prüfsensor an einer von der Schlauchmündung entfernten Zwischenstelle liegt. Der weiter innen gelegene Schlauchteil schützt demnach einen permanent an einer Zwischenstelle des Schlauchs angebrachten Prüfsensor vor direkter Verschmutzung. Ferner ist für Temperaturkonstanz gesorgt. Bei einem Wechsel des Sensors bleibt der Bauchraum unbeeinträchtigt.

Gemäß einem alternativen Verfahren kann die Körperflüssigkeit natürlich nach außerhalb des Körpers abgesaugt und dort analysiert werden.

Ferner wird bevorzugt eine Mikrodialysesonde verwendet, mit der die Stoffe in der Körperflüssigkeit entnommen werden.

Die erfindungsgemäße Vorrichtung zur Erfassung der Konzentration bzw. des Vorhandenseins von Stoffen in Körperflüssigkeiten weist einen permanent im Körper implantierten Zugang zum Körperinneren auf, der zur Analyse der Flüssigkeiten so angepaßt ist, daß über diesen Körper auf die Flüssigkeiten zugegriffen werden kann.

Vorzugsweise ist der Zugang eine permanent implantierte Vorrichtung zur Verabreichung von Arzneimitteln, insbesondere ein in der Haut implantierter Portkörper mit einer sich ins Körperinnere erstreckenden Schlauchanordnung.

Der Zugang der erfindungsgemäßen Vorrichtung bzw. der Portkörper weist bevorzugt Durchgänge auf, mittels derer ein Prüfsensor in das Körperinnere eingeführt wird. Bevorzugt hat der Zugang bzw. der Portkörper einen separaten Schlauch zur Einführung eines solchen Prüfsensors.

Eine besonders bevorzugte Ausgestaltung der erfindungsgemäßen Vorrichtung ist dadurch gekennzeichnet, daß dem Zugang eine Mikrodialysesonde zugeordnet ist, über die die Stoffe in der Körperflüssigkeit entnommen werden.

Die Erfindung betrifft nicht die Verwendung eines permanent im Körper implantierten Zugangs zum Körperinneren zur Erfassung der Konzentration bzw. des Vorhandenseins von Stoffen in Körperflüssigkeiten. Bei einer solchen Verwendung können alle oben aufgeführten vorteilhaften Ausführungsformen der erfindungsgemäßen Vorrichtung realisiert werden.

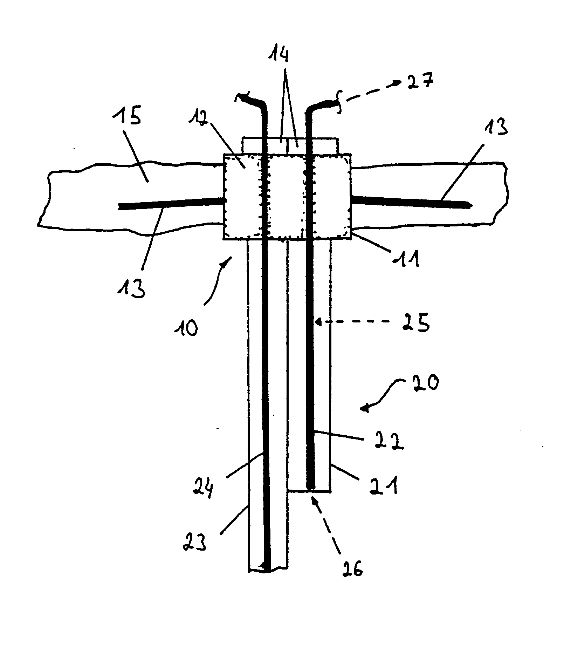

Die Erfindung wird im weiteren anhand der beiliegenden Figur näher erläutert, die in schematischer Darstellung einen erfindungsgemäß angepaßten, implantierten Portkörper zeigt.

Der Portkörper, der in der Figur in der Gesamtheit mit 10 bezeichnet ist, ist in einem in der Haut 15 implantierten Zustand gezeigt. Er besteht aus einem Schaftteil 11, an dem außen ein in etwa scheibenförmig ausgestalteter Verankerungsteil 13 befestigt ist, der zur Fixierung des Portkörpers 10 in der Haut 15 dient.

Der Schaftteil 11 bildet ein hohles Gehäuse, in dem eine elastische selbstschließende Membran 12 aufgenommen wird.

Am unteren Ende erstrecken sich vom Portkörper 3 getrennte Schläuche weg, nämlich ein Zufuhrschlauch 23, der in der Figur nicht in seiner gesamten Länge dargestellt ist, sowie ein kürzerer Ansaugschlauch 21. Durch die Membran 12 hindurch kann in jeden der Schläuche 23 und 21 jeweils ein Katheter eingeführt werden, nämlich im vorliegenden Beispiel der Zuführkatheter 24 und der Ansaugkatheter 22.

Durch den Zufuhrkatheter 24 wird eine Stelle an der Mündung des Schlauches 23 mit einem Medikament, beispielsweise mit Insulin, versorgt, während durch den Saugkatheter 22 von einer Stelle am Ende bei der Mündung des Schlauches 21 Körperflüssigkeit entnommen werden kann.

Schematisch sind auf der Oberseite des Schaftes 11 noch Befestigungsabschnitte 14 vorgesehen, die zur Verbindung von nicht dargestellten oberen Dicht- bzw. Abschlußdeckeln an den Kathetern 24 und 22 vorgesehen sind.

Der Zufuhrschlauch 23 wird bevorzugt genormte Längen von 150 bzw. 180 mm aufweisen, während der Saugschlauch 21 etwa eine Länge von wenigstens 30, vorzugsweise von 60 bis 120 mm, gegebenenfalls bis 180 mm annehmen kann. Anhand der hier dargestellten Anordnung wird nunmehr ersichtlich, daß dieser Portkörper sowohl zur Zufuhr von Arzneimitteln über den Zufuhrkatheter 24 als auch zum Zugriff auf Körperflüssigkeit über den Saugkatheter 22 verwendet werden kann. Bevorzugt erstrecken sich die Schläuche 23 und 21 in den Bauchraum des Patienten hinein, wo sich die Zellflüssigkeit befindet, der entweder eine Arznei zugeführt werden muß oder die zur Prüfung bestimmter Konzentrationen analysiert werden soll.

Mit gestrichelten Pfeilen sind nun die verschiedenen möglichen Meßorte für Konzentrationsmessungen oder zum Messen des Vorhandenseins bestimmter Stoffe angedeutet. An der Meßstelle 25 befindet sich in dem Fall ein Sensor, wo mittels eines Unterdrucks Flüssigkeit von der unteren Mündung des Katheters 22 etwas nach oben gesaugt wird, um gerade an dieser Zwischenstelle 25 eine Messung vorzunehmen. Wie schon oben beschrieben, ist der hier permanent an der Stelle 25 angebrachte Sensor durch den unteren Abschnitt des Schlauches 21 bzw. des Katheters 22 vor Ablagerungen geschützt.

Wenn die Messung an einer Stelle erfolgt, an der keine zu starken Ablagerungen zu befürchten sind, kann der Prüfsensor ebenfalls an der Stelle permanent angebracht werden, die mit dem gestrichelten Pfeil 26 bezeichnet ist. Der Vorteil dieses Erfassungsverfahrens liegt darin, daß keine Ansaugung mehr nötig ist und die Erfassung direkt an einem vorgesehenen Meßpunkt ermöglicht wird.

Bei den beiden vorbeschriebenen Ausführungsformen, in denen der Prüfsensor im Schlauch bei 25 bzw. am Schlauchende 26 permanent verbleibt, wird ein elektronischer Prüfsensor verwendet, dessen Anschlußdrähte mit dem Katheter 22 aus dem Körper herausgeführt werden können.

An dieser Stelle soll noch bemerkt werden, daß solche elektrischen Prüfsensoren meist eine Arbeitselektrode, eine Gegenelektrode sowie eine stromlose Referenzelektrode aufweisen. Wenn der Schaft 11 des Portkörpers (wie es üblicherweise der Fall sein wird) aus einem metallischen Material besteht, kann erfindungsgemäß vorteilhafterweise auch der Schaft 11 als Referenzelektrode oder Gegenelektrode genutzt werden, so daß sich bei solchen Messungen erfindungsgemäß auch eine Vereinfachung des elektrischen Sensors erreichen läßt.

Eine weitere Möglichkeit, die Anordnung, die in der Figur dargestellt ist, zu nutzen, besteht darin, durch den Schlauch 21 zur einmaligen Messung oder für jede Einzelmessung lediglich einmal eine Sonde einzuschieben, die an ihrem vorderen Ende einen Teststreifen zur Konzentrationserfassung aufweist. Die Meßwertbestimmung und - auswertung kann im eingeschobenen Zustand oder nach Entfernen nach bekannten Verfahren erfolgen.

Schließlich besteht eine sehr einfache Art der erfindungsgemäßen Erfassungstechnik darin, über den Katheter 22 ganz einfach Flüssigkeit aus dem Körperinneren vom Meßort weg nach außen zu saugen, wie durch den gestrichelten Pfeil 27 dargestellt wird. Die so erhaltene Körperflüssigkeit kann dann auf bekannte Weise im Labor analysiert werden.

## Patentansprüche

1. Vorrichtung zur Erfassung der Konzentration bzw. des Vorhandenseins von Stoffen in Körperflüssigkeiten, mit einem in die Haut implantierbaren Portkörper (10), mit einem Zufuhrschlauch (23) und einem davon getrennten Ansaugschlauch (21), welche sich vom Portkörper (10) weg erstrecken, wobei der Ansaugschlauch (21) ein Zugang zum Körperinneren ist der zur Analyse der Flüssigkeiten so angepasst ist, dass über diesen Körperflüssigkeit entnommen werden kann.

2. Vorrichtung nach Anspruch 1, **dadurch gekennzeichnet, dass** der Zugang (21) bzw, der Portkörper (10) Durchgänge ausweist, mittels derer ein Prüfsensor in das Körperinnere einführbar ist.

3. Vorrichtung nach einem der Ansprüche 1 oder 2, **dadurch gekennzeichnet, dass** dem Zugang (21) eine Mikrodialysesonde zugeordnet ist, über die die Stoffe in der Körperflüssigkeit entnehmbar sind.

## Claims

1. A device for detecting the concentration and/or presence of substances in body fluids, comprising: a port body (10) which can be implanted into the skin; a flexible supply tube (23); and a flexible suction tube (21) which is separate from the flexible supply tube and extends away from the port body (10), wherein the flexible suction tube (21) is an access to the interior of the body and is adapted for analysing the fluids, such that body fluid can be removed via it.

2. The device according to claim 1, **characterised in that** the access (21) and/or port body (10) comprises passages by means of which a testing sensor can be introduced into the interior of the body.

3. The device according to any one of claims 1 or 2, **characterised in that** a micro-dialysis probe is assigned to the access (21), and the substances in the body fluid can be removed via the micro-dialysis probe.

## Revendications

1. Système de détection de la concentration et/ou de la présence de substances dans les liquides de l'organisme, doté d'un port (10) implantable dans la peau, avec un tuyau souple d'amenée (23) et un tuyau souple d'aspiration (21) distinct de celui-ci, lesquels s'étendent à partir du port (10), le tuyau souple d'aspiration (21) étant un accès à l'intérieur de l'organisme qui peut être adapté pour une analyse des liquides de sorte que du liquide de l'organisme puisse être prélevé via celui-ci.

2. Système selon la revendication 1, **caractérisé en ce que** l'accès (21) et/ou le port (10) comprend des passages au moyen desquels un capteur de contrôle peut être introduit à l'intérieur de l'organisme.

3. Système selon la revendication 1 ou 2, **caractérisé en ce qu'**une sonde de microdialyse est affectée à l'accès, via laquelle les substances peuvent être prélevées dans le liquide.
